# EUROPEAN PATENT SPECIFICATION

(11) **EP 4 525 762 B1**
(45) Date of publication and mention of the grant of the patent: **01.07.2026**
(21) Application number: 23729504.3
(22) Date of filing: 17.05.2023
(51) Int. Cl.: A61B 18/20

(54) **HANDPIECE FOR THE TREATMENT OF SKIN BY LIGHT RADIATION**
HANDSTÜCK ZUR BEHANDLUNG DER HAUT MIT LICHTSTRAHLUNG
PIÈCE À MAIN POUR LE TRAITEMENT DE LA PEAU PAR RAYONNEMENT LUMINEUX

(30) Priority: 19.05.2022 IT 202200010370
(43) Date of publication of application: 26.03.2025
(73) Proprietor: EL.EN. S.p.A., 50041 Calenzano (FI) (IT)
(72) Inventor: TAGLIAFERRI, Marco, 50041 Calenzano (FI) (IT); TUSI, Daniele, 50041 Calenzano (IT); BRUGIONI, Alfredo, 50041 CALENZANO (IT); BALDINI, Andrea, 50041 CALENZANO (IT); MARGHERI, Fabrizio, 50041 CALENZANO (IT)
(74) Representative: Mannucci, Michele
(86) International application number: PCT/IB2023/055057
(87) International publication number: WO 2023/223221

(56) References cited:
- WO-A1-2005/077459
- WO-A1-2018/185773
- KR-A- 20210 018 636
- US-A- 5 486 172
- US-A- 6 059 820
- US-A1- 2003 065 314
- US-A1- 2006 271 028

## Description

### Technical field

The present invention concerns the field of machines for aesthetic and dermatological treatment by means of light radiation produced, for example, by laser sources, LED sources, or other light sources. More in particular, the invention relates to a handpiece applicator of light energy, and to an aesthetic and dermatological treatment device utilizing the handpiece. The invention also relates to a method for cooling the handpiece during a non-therapeutic and non-surgical procedure for treating skin.

### Background art

As is known, in recent years treatment of the skin by means of laser has increased considerably, both for curative purposes, for example to treat vascular lesions, hyperpigmentation, benign pigmented lesions and scars, and for purely aesthetic treatment purposes, for example, reduction of wrinkles and removal of hair and, more in general, improvement of the appearance of the skin.

According to the type of treatment, it is necessary to use a laser with a suitable wavelength and specific emission parameters to obtain, for example, an irreversible but selective damage on the hair follicles in the removal of hair, or coagulation of small vessels or destruction of accumulations of melanin, to remove cutaneous or vascular lesions.

Per applications in the dermatological field, it must be borne in mind that different wavelengths interact in different ways with the skin, at the level of reflection, absorption and penetration, as a function of the patient's phototype, i.e., of the amount di melanin. The field of applications and performances of a laser device can therefore be restricted and partially limited by the type of source, which in turn requires specific and further differentiated accessories based on the operating parameters to be used.

In general, a laser device for these types of treatments is provided with a laser source located inside a transportable platform, and a laser emission terminal, provided with a suitable optical device for manipulation of the laser light beam capable of producing a suitable distribution of intensity on the surface of the skin, both in terms of shape and size and in terms of uniformity. This terminal is provided in a handpiece and connected to the source by means of a waveguide, for example an optical fibre cable, or similar, which allows the laser radiation to be conveyed to the patient in the form of light spot, for example, but not exclusively, with a circular shape. The handpiece/terminal is generally removable from the connection cable to the source.

Some types of treatment, such as hair removal, require relatively high exposures and fluences of laser radiation and the handpieces most commonly available to date must be placed statically on the part of the subject to be treated and moved to contiguous positions after each emission, until the whole of the area involved has been covered.

The treatment generates considerable heat. For this reason, the handpieces are associated with systems for cooling the epidermis. In this context, reference is made to document WO2018/185773 A1.

A first cooling system currently in use, for example, uses a cold air ejector connected, by means of a pipe, to a cooling circuit and to a compressor, provided in the platform of the treatment device or produced in an external cooling device. This ejector is integrated in a handle that forms the handpiece and to which the laser emission terminal is reversibly attached.

The handpiece is held at an operating distance from the skin through a spacer. The cold air is conveyed onto the area of the skin to be treated, which is thus cooled due to air-skin heat exchange. After the laser pulse has been applied, the handpiece is moved to the nearby area, already cooled by the air, which covers a larger surface with respect to the area covered by the laser beam.

Another type of cooling currently in use uses a jet of a highly volatile substance (for example, a coolant) in liquid state, directed onto the area to be cooled; this substance evaporates rapidly in contact with the epidermis, producing the desired cooling effect in the instant prior to irradiation/treatment; said type of cooling uses a handpiece associated with a cooling module containing a cooling fluid in liquid state and a pressurized gas source that maintains the fluid in liquid state before directing it onto the skin, where it expands in a cooling spray directed toward the area of epidermis to be cooled. An example of this device is disclosed in US2008/0188840.

Another type of laser handpiece with integrated cooling system is disclosed, for example, in WO2019077492. This document describes a handpiece for laser treatments that act in greater depth with respect to the dermatological/aesthetic treatments described above. It has a handpiece provided with the removable laser terminal and with a cooling plate to be placed in contact with the skin (technique defined as contact cooling). This cooling plate is cooled by means of a system of Peltier cells and this system is associated with a cooling circuit connected to the handpiece via a pipe and present on the mobile platform. In devices that use the contact cooling technique, the skin is cooled by conduction, rather than by convection as occurs in the cases mentioned previously.

Another type of handpiece that uses a conduction cooling body, in direct contact with the skin, is disclosed, for example, in WO2021/094938. This handpiece is provided with a sapphire window to be placed in contact with the epidermis to be cooled. The window is transparent to the laser radiation that produces the treatment on the epidermis. This window is surrounded by a cooling frame. The body of this frame is provided internally with ducts containing a cooling liquid, which removes heat from the frame and this removes it from the cooling body, which in turn cools the epidermis.

US20170304645 discloses a handpiece provided with a sapphire window, through which the laser radiation passes to carry out the treatment on the epidermis. The window is adapted to come into contact with the epidermis. To cool the epidermis, the window is cooled by a cooling liquid. In particular, the window is provided with channels obtained completely inside the body of the sapphire; these channels are connected to a cooling liquid circulation circuit.

As mentioned, a problem related to the handpieces described above is linked to the fact that the treatment devices with which the handpieces are associated are used for different purposes and thus require different laser sources and optical handling or beam "reshaping" systems. In currently known devices, mobile platforms have more than one laser source, among which the most suitable source is selected, according to requirements. Instead, the laser radiation emission terminals associated with the handpieces must be replaced as a function of the optics that are to be used.

Therefore, it is necessary to have a respective laser emission terminal available for each type of treatment, i.e., it is necessary to have a large set of laser terminals, also considering the need for spare terminals to replace any damaged terminals. It is clear a that the greater the number of terminals is, the greater the costs for all the equipment required for the different treatments will be.

Moreover, the operation to replace of the terminals is not that simple and requires some attention.

Furthermore, the laser emission terminals are subject to inevitable heating due to the reflection of the laser radiation by the skin toward said handpieces. The cooling systems currently used are not capable of adequately cooling the handpieces too and in certain parts of the handpiece the temperature may become very high for continual use by the operator.

### Summary

The invention is defined in the appended set of claims. The object of the present invention is to solve the problems related to handpieces of aesthetic and/or dermatological devices of known type for treating the skin by means of light radiation.

Therefore, an important object of the present invention is to produce a handpiece for aesthetic and/or dermatological devices for treating the skin by means of light radiation that can be used, at least in part, for different types of treatment.

Another important object of the present invention is to produce a handpiece for aesthetic and/or dermatological devices for treating the skin by means of light radiation that allows optimal cooling of the part to be treated.

Yet another important object of embodiments of the present invention is that of producing a handpiece for aesthetic and/or dermatological devices for treating the skin by means of light radiation that allows adequate cooling of said handpiece.

One more important object of embodiments of the present invention is to produce a handpiece for aesthetic and/or dermatological devices for treating the skin by means of light radiation that is easy to use.

These and other objects, which will more apparent below, are achieved with a handpiece for treating the skin by means of light radiation, wherein the handpiece comprises a gripping structure, a seat for an interchangeable optical assembly, and a cooling device of the epidermis; wherein the cooling device comprises:
- a cooling body of the epidermis adapted for a light beam for treating the skin to pass through, the cooling body being provided with an outer surface comprising:
   ▪ a rear entrance surface of the light beam;
   ▪ an opposite front surface for contact with the epidermis and for exit of the light beam; and
   ▪ a lateral surface provided between the front surface and the rear surface;
- a sleeve, in which the cooling body is housed; wherein the sleeve comprises an inner surface at least partially surrounding the lateral surface of the cooling body;
- a circulation chamber of a cooling fluid defined between the inner surface of the sleeve and at least one portion of the outer surface of the cooling body.

Advantageously, the circulation chamber of a cooling fluid is defined, at least in part, between the inner surface of the sleeve and the lateral surface of the cooling body and at least partially surrounds the cooling body.

The cooling fluid is adapted to cool the cooling body. Advantageously, the cooling fluid flows over the lateral surface of the cooling body, i.e., is in direct contact therewith when it is in the circulation chamber.

The circulation chamber is a space defined at least in part by the lateral surface of the cooling body and by the inner surface of the sleeve. The cooling fluid is therefore in contact with at least part of the lateral surface of the cooling body.

The handpiece comprises a first supply duct of the cooling fluid to the cooling fluid circulation chamber and a second delivery duct of the cooling fluid from the cooling fluid circulation chamber, adapted to operatively connect the handpiece to a cooling fluid cooling circuit.

To summarize, the handpiece for treating the skin through light radiation comprises a gripping structure, a seat for an interchangeable optical assembly and a cooling device of the epidermis, this latter comprising a cooling body of the epidermis adapted for a light beam for treating the skin to pass through. The cooling body is provided with an outer surface comprising a rear entrance surface of the light beam, an opposite front surface for contact with the epidermis and for exit of the light beam, and a lateral surface extending between the front surface and the rear surface. The cooling device further comprises a sleeve, in which the cooling body is housed. The sleeve comprises an inner surface at least partially surrounding the lateral surface of the cooling body. A circulation chamber of a cooling fluid that at least partially surrounds the cooling body is defined between the inner surface of the sleeve and at least one portion of the outer surface of the cooling body.

An optical assembly is for example, an optical assembly that allows focusing of the radiation, or an optical assembly for divergence or a diffractive/refractive array, or yet other optical assemblies.

Advantageously, in some embodiments, the inner surface of the sleeve extends from the cooling fluid circulation chamber toward the rear surface of the cooling body.

As better explained below, the arrangement of a circulation chamber in direct contact with the cooling body and advantageously close to the front surface of the cooling body and at a distance from the rear surface, makes it possible to obtain optimal cooling of the surface of the cooling body in contact with the epidermis during treatment, avoiding excessive cooling of the rear surface and hence the formation of condensation on the rear surface.

In general, the handpieces can be provided with
- a fixed optical assembly, which is assembled in the handpiece during construction of said handpiece and wherein the optical assembly is selected based on the type of treatment to be carried out and cannot normally be changed except for repair operations, with complex disassembly of the handpiece (if the operator requires an optical assembly of different type, it is necessary to take a handpiece with the suitable optical assembly from a magazine), or, as in the case of the present invention,
- an interchangeable optical assembly, which can be selected in a kit of different optical assemblies available to the operator of the treatment before the treatment step, in order to use the most suitable optical assembly. The operator selects the most suitable assembly and rapidly replaces the one associated with the handpiece with it. Interchangeability is simple and is, for example, carried out by conventional coupling/connection means, for example, sliding and snap fastening, screw on, bayonet coupling, etc.

A confined environment is defined between the rear surface of the cooling body and the optical assembly. In handpieces provided with interchangeable optical assemblies, during replacement of one optical assembly with another, this environment is exposed to the air. This air contains, in general, depending on the areas in which the handpiece is used, more or less humidity. When the optical assembly is changed, the humid air remains trapped between optical assembly and cooling body. During use, the cooling body is cooled by the cooling fluid and this cooling, if not suitably managed, can cause condensation of the water contained in the air on the rear surface of the cooling body. The droplets of condensation in practice form lenticular bodies that focus the light beam directed toward the epidermis, i.e., modify the distribution of the intensity of the light beam, which can thus cause localized burns on the epidermis, as well as damages to the cooling body.

In relation to this problem of condensation, providing a numerical example, the amount of air present in the confined environment between cooling body and interchangeable optical assembly can, for example, have a volume of 13 cm³ (variable according to the optical configuration of the optical assembly, and hence of the axial distance between the rear surface of the cooling body and the optical surface of the optical assembly close to the cooling body. The maximum amount of water contained in a volume of air is regulated by psychrometric diagrams and is equal to around 24g per m³ of air, when the air is at atmospheric pressure and a temperature of 25°C. In these conditions the relative humidity is equal to 100%. A representative condition of use of the handpiece in conditions of high humidity can, for example, be with air at 22°C, with relative humidity RH=80%. In this condition, the aforesaid volume of air contains around 0.31 mg of water. In the presence of condensation on the rear surface of the cooling body, the amount of water is sufficient to disturb the light radiation passing through the cooling body. The disturbance effect can vary and depends on the condensation process; as already mentioned, it can lead to the formation of diffusive areas or to the formation of lenticular bodies on the cooling body capable of permanently altering the distribution of incident intensity. It must also be mentioned how the removable nature of the handpiece leads to continuous changes of air inside said chamber, with possible formation of local accumulations of water on the rear surface of the cooling body due to the aforesaid condensation processes.

According to the present invention, by concentrating cooling around the cooling body, it is possible to concentrate cooling close to the front surface of the cooling body, and to use a cooling fluid at a temperature that is not overly low. At the same time, the distance between the cooling fluid circulation chamber and the rear surface of the cooling body, together with the high thermal resistance of the cooling body, means that, during use, a temperature gradient increasing from the front surface toward the rear surface is established along the axial extension of the cooling body. Consequently, the latter will be at a higher temperature than the front surface, such as to avoid condensation of humidity on said rear surface.

In preferred embodiments, to obtain an effective heat exchange, the cooling fluid is a liquid.

In preferred embodiments, the cooling body is made
- in one piece, or
- in at least two pieces arranged facing one another in the direction of passage of the light beam and made of the same material or of a different material, wherein the piece on which the front surface is present can have a lower coefficient of thermal conductivity with respect to the coefficient of thermal conductivity of the other piece.

In preferred embodiments, the cooling fluid circulation chamber is defined, at least in part, between the inner surface of the sleeve and the lateral surface of the cooling body and at least partially surrounds the cooling body. Preferably, the lateral surface of the cooling body extends from the front surface to the rear surface.

The advantageous presence of a cooling fluid circulation chamber that surrounds the cooling body close to the front surface of the cooling body allows optimal heat exchange between cooling fluid and cooling body, which is particularly uniform around the latter.

According to some embodiments, when the cooling body is made in two pieces or portions, arranged side by side and in sequence along the path of the light beam, the cooling fluid circulation chamber can be defined at least in part between the two pieces.

Preferably, the cooling body, transparent to light radiation, has a limited coefficient of thermal conductivity, in order to promote establishment of the thermal gradient between front surface and rear surface. For example, the cooling body can have a coefficient of conductivity of less than 50 W/mK.

The limited coefficient of thermal conductivity and the arrangement of the cooling fluid circulation chamber close to the front surface of the cooling body means that heat removal is concentrated in the area of the front surface of the cooling body, and becomes substantially less efficient in the areas closer to the rear surface.

For example, the cooling body can comprise sapphire, "fused silica", optical glass or other materials with similar optical and thermal conductivity properties.

The cooling body can be cylindrical, conical, prismatic in shape, etc., with two end surfaces or faces, or bases, preferably parallel to each other, for example circular, ellipsoidal, quadrangular, or more generally, polygonal in shape.

The cooling body is in practice a treatment window di through which the light radiation passes, which being in direct contact with the area to be treated, allows particularly effective cooling of the epidermis to be obtained due to the fact that the heat is removed by conduction directly from the area of the epidermis affected by the light beam. At the same time, the handpiece thus configured is particularly compact.

In preferred embodiments, the cooling fluid circulation chamber is positioned, at least in part, between the front surface of the cooling body and a median plane, equidistant from the front surface and from the rear surface of the cooling body.

More in general, preferably, the circulation chamber extends prevalently closer to the front surface of the cooling body with respect to the rear surface of said body.

This positioning of the cooling fluid circulation chamber allows a sufficiently cooled fluid to be carried to an area of the cooling body close to the front surface of the body that is to come into contact with the epidermis.

Preferably, considering the median plane parallel to the front surface and to the rear surface of the cooling body, such as to be equidistant from the front surface and from the rear surface of the cooling body, the circulation chamber comprises an inlet opening of the cooling fluid located between the front surface of the cooling body and said median plane; preferably the circulation chamber further comprises an outlet opening of the cooling fluid located between the front surface of the cooling body and the median plane.

Preferably, considering the median plane parallel to the front surface and to the rear surface of the cooling body, such as to be equidistant from the front surface and from the rear surface of the cooling body, the cooling fluid circulation chamber is positioned entirely between the front surface of the cooling body and said median plane.

In preferred embodiments, the cooling body has a greater axial dimension with respect to a transverse dimension, i.e., a dimension orthogonal to the axial dimension. Axial dimension is meant as the dimension in the direction of propagation of the light beam through the cooling body. This means substantially that the distance between the two front and rear surfaces of the cooling body is greater than the distance between the farthest away lateral surfaces of the body in the case of a prismatic body, or in the case of a body with circular or ellipsoidal cross section, it is greater than the diameter or than the major axis of the cross section.

This increase of the axial dimension with respect to the transverse dimension allows better control of the temperature of the rear surface of the cooling body, preventing it from becoming too cool, i.e., from being too close to the front surface, where the majority of cooling takes place.

In preferred embodiments, the sleeve comprises a first portion of the inner surface surrounding the cooling fluid circulation chamber and a second portion of the inner surface extending from the cooling fluid circulation chamber toward the rear surface of the cooling body. The second portion of inner surface has an extension in axial direction greater than the first portion of inner surface, preferably at least double that of the first portion of inner surface.

In use, the light radiation reflected and backscattered from the skin affects the inner surface of the sleeve in which the cooling body is enclosed. The inner surface of the sleeve thus defines a collection and reflection surface of reflected and backscattered light radiation, which tends to heat the second portion of surface of the sleeve, where no cooling fluid circulates, causing an increase of the temperature gradient between front surface and rear surface of the cooling body and obtaining a rear surface at a temperature substantially higher with respect to the temperature of the front surface.

According to preferred embodiments, a heat conduction interface between the inner surface of the sleeve and the cooling body is defined between the cooling fluid circulation chamber and the rear surface of the cooling body. For example, said heat conduction interface comprises a further chamber containing cooling fluid. Preferably, this further chamber is in fluid communication with the cooling fluid circulation chamber through a more or less annular passage. Preferably, the further chamber has an annular cross section, of smaller radial dimension with respect to the radial dimension of the cooling fluid circulation chamber.

In practice, in this further chamber the cooling fluid is in static or quasi-static conditions, or in any case moves with a very low speed with respect to the inflow speed of the fluid into the circulation chamber. In this further chamber, which can be considered also as a "stilling chamber", the cooling fluid enters, for example, at the first use, drawn through the passage. In said further chamber the fluid in practice acts as "filling material" with known heat exchange properties, allowing the temperature along the cooling body to be controlled. In practice, the fluid in the further chamber forms a medium of thermal transmission by conduction that facilitates the transmission of heat from the inner surface of the sleeve to the cooling body. In this way, the heat generated by the light radiation reflected and backscattered from the skin during treatment and that affects the inner surface of the sleeve is transferred to the cooling body, in the volume of the latter between the circulation chamber and the rear surface. Thermal conduction through the fluid trapped in the further chamber is thus a factor that contributes in establishing the temperature gradient between front surface (at minimum temperature) and the rear surface (at maximum temperature) of the cooling body.

In some embodiments, the heat conduction interface between the inner surface of the sleeve and the cooling body can be made of a thermally conductive liquid material, different from the cooling liquid, for example diathermic oils. Moreover, in place of the liquid material, the heat conduction interface can be made of a thermally conductive element in solid form, for example, in the form of a quill or sheath. These or other thermally conductive materials can be arranged in said further chamber described above, in place of the cooling fluid.

When wishing to further increase the temperature gradient along the axial extension of the cooling body, in order to have on the rear face thereof an even higher temperature, one or more resistor elements can be installed, through which electrical current passes and which produce heat. For example, one or more heating elements can be located between the lateral surface of the cooling body and the inner surface of the sleeve, close to the rear surface. In other embodiments, one or more resistor heating elements can be applied in laminar form on the rear surface. In this case, the resistor elements will be suitably transparent to the light radiation that must pass through the cooling body.

According to preferred embodiments, the handpiece further comprises an interface integral with the sleeve and coaxial thereto. The interface forms a connection for the interchangeable optical assembly. Preferably, the interface comprises a bushing with one end fixed to the sleeve, preferably through a thermally conductive coupling, preferably a screw coupling.

In some embodiments, the bushing comprises an inner annular boss, in practice defining a diaphragm, which forms a collection surface of light radiation reflected and backscattered from the skin during use of the handpiece. The surface of the annular boss tends to heat due to the backscattered light radiation and conducts heat toward the rear surface of the cooling body causing a heating thereof.

The bushing is preferably made of a conductive material, with a high coefficient of thermal conductivity. This feature, together with strengthening of the thermal bridge between bushing and sleeve, ensures that most of the heat generated by the light radiation backscattered and collected from the bushing is transmitted to the sleeve limiting the amount of heat that instead heats the volume of air contained inside the bushing.

According to preferred embodiments, the handpiece comprises a first supply duct of the cooling fluid to the cooling fluid circulation chamber and a second delivery duct of the cooling fluid from the cooling fluid circulation chamber.

According to preferred embodiments, the optical assembly comprises a connection interface adapted to be connected to a transmitter of luminous power, for example, a fibre or tube waveguide. In this case, the light radiation source can be located in a base of an apparatus, to which the handpiece and the optical assembly applied thereto are connected.

In other embodiments, the light radiation source can be housed in the optical assembly or in the handpiece. In this case, the light radiation source will be connected to the base of the apparatus by an electrical power cable.

The light radiation that can be used can, for example, be a coherent radiation produced by a laser source and therefore the light beam is a laser beam, with characteristics of power and wavelength related to the type of treatment to be carried out. The laser source can be housed in a base of an apparatus, with which the handpiece is interfaced and transmitted through a waveguide, for example of fibre, hollow guide or articulated arm with mirrors type.

In other embodiments the laser radiation can be generated by one or more laser diodes housed in the or supported by the handpiece, supplied by the base of the apparatus through an electrical power supply cable.

In other embodiments, the light radiation can be incoherent and can, for example, be produced by one or more LEDs or other light sources. Also in this case, the light source can be housed in the base of the apparatus and conveyed toward the handpiece through, for example, a system of mirrors. In other embodiments, the incoherent light radiation source can be housed in the handpiece or in the interchangeable optical assembly. In this case, the source is supplied by an electrical connection cable to the base of the apparatus.

According to some embodiments, the handpiece comprises an electronic reference for identification of the specific optical assembly inserted in said seat.

According to another aspect, the invention also relates to a device for treating the skin by means of light radiation comprising a base platform provided with an electronic control unit of the device, a cooling circuit for a cooling fluid and at least one power source, wherein
- the power source is adapted to be operatively connected, through a power conveying duct, to a handpiece according to one or more of the embodiments or configurations described above;
- the cooling circuit is adapted to be operatively connected to the handpiece by means of a first pipe for supplying cooling fluid to the handpiece from the cooling circuit, and a second pipe for return of cooling fluid from the handpiece to the cooling circuit;
- the electronic control unit is adapted to be operatively connected with the handpiece by means of electrical wiring.

For example, the power source is a laser source and the power conveying duct is an optical fibre, a waveguide or similar capable of transporting the laser radiation.

For example, the power source is an electrical power supply adapted to supply one or more LEDs located in the handpiece and adapted to produce the light radiation for treatment; the power conveying duct is an electrical duct.

The treatment device further comprises a kit of different optical assemblies that can be used with the handpiece.

According to another aspect, the invention further concerns a method for cooling a handpiece for treating the skin by means of light radiation, wherein the handpiece comprises a gripping structure and a seat for an interchangeable optical assembly, and a cooling device of the epidermis; wherein the cooling device comprises:
- a cooling body of the epidermis adapted for a light beam for treating the skin to pass through, the cooling body being provided with an outer surface comprising:
   a rear surface for entry of the light beam;
   an opposite front surface for contact with the epidermis and for exit of the light beam; and
   a lateral surface extending between the front surface and the rear surface;
and wherein the handpiece comprises a confined environment facing the rear surface of the cooling body and delimited at least in part by the optical assembly.

The method provides for, during emission of light radiation through the cooling body, conditioning of the temperature on the rear surface of the cooling body to a value greater than the dew point temperature relating to the space in the confined environment facing the rear surface of the cooling body.

Preferably, the rear surface of the cooling body is conditioned to a temperature greater than 20°C and more preferably between 20°C and 40°C.

Preferably, the light radiation emitted by the handpiece has a power between 20W and 100W; preferably the light radiation is a laser radiation.

Preferably, the cooling device of the handpiece further comprises
- a sleeve, in which the cooling body is housed; wherein the sleeve comprises an inner surface at least partially surrounding the lateral surface of the cooling body;
- a circulation chamber of a cooling fluid defined between the inner surface of the sleeve and at least one portion of the outer surface of the cooling body,

Conditioning of the temperature of the rear surface further provides for cooling of the cooling body by means of cooling fluid circulation in the circulation chamber.

Preferably, conditioning of the temperature of said rear surface further provides for heating of the walls of said confined environment facing the rear surface of the cooling body by means of
thermal conduction of heat coming from the sleeve heated by the light radiation reflected and backscattered from the skin toward the inside of the handpiece and
the light radiation reflected and backscattered from the skin directly against the walls of the confined environment facing the rear surface of the cooling body.

Preferably, the handpiece further comprises an interface integral with the sleeve and coaxial thereto; wherein the interface forms a connection for the interchangeable optical assembly.

Preferably, the interface integral with the sleeve comprises a bushing with one end fixed to the sleeve, preferably through a thermally conductive coupling, preferably a screw coupling; preferably, the bushing comprises an inner annular boss defining a collection surface of light radiation backscattered from the skin during use of the handpiece.

Preferably, the cooling fluid circulation chamber is positioned, at least in part, between the front surface of the cooling body and a median plane, equidistant from the front surface and from the rear surface of the cooling body, and wherein removal of heat by the cooling fluid takes place prevalently in the part of cooling body between the front surface of the cooling body and said median plane.

### Brief description of the drawings

The invention will be better understood by following the description and the accompanying drawings, which illustrate a non-limiting example of embodiment of the invention. More in particular, in the drawing:
Fig. 1 represents a schematic view of a device for treating the skin according to the invention;
Fig. 2 represents an axial sectional view of a handpiece according to the invention, sectioned along the line II-II of Fig. 5;
Fig. 3 represents a longitudinal sectional view, according to a plane of a front portion of the handpiece, sectioned along the line III-III of Fig. 4;
Fig. 4 represents a cross sectional view of the handpiece of the preceding figures, sectioned along the line IV-IV of Fig. 2, relating to the circulation chamber;
Fig. 5 represents a cross sectional view of the handpiece of the preceding figures, sectioned along the line IV-IV of Fig. 2, relating to a further circulation chamber.
Figs. 6a and 6b represent axial and transverse schematic sections of the portion of handpiece relating to the cooling body highlighting the different levels of temperature reached in the various parts;
Fig. 7 represents a graph showing the temperature measured on the external extrados of the handpiece, as a function of the time,
Fig. 8 represents a graph of the temperature in the points of interest 1-4 on the external extrados of the handpiece, as a function of laser power;
Fig. 9 represents a longitudinal sectional view analogous to that of Fig. 3, relating to a first variant of handpiece;
Fig. 10 represents a longitudinal sectional view analogous to that of Fig. 3, relating to a second variant of handpiece.

### Detailed description of embodiments

With reference to the aforesaid figures, a device for treating the skin by means of light radiation according to the invention is indicated as a whole with the number 1000. This device comprises a base platform 100 with which a handpiece 10 is operatively connected.

In this example, the light radiation is a laser radiation.

In general, in the present context, light radiation is meant as a coherent electromagnetic radiation in a range of wavelengths in the visible or in the near infrared, preferably between 400nm and 3000nm.

The platform 100, described here briefly, comprises internally two laser sources 101 and 102, a cooling circuit 103 and an electronic control unit of the device 104.

For example, the two laser sources 101 and 102 are of "free running long pulsed" type with active medium, known per se, such as an Nd:YAG source (at 1064 nm) and an Alexandrite source (at 755 nm).

A waveguide 105, for example comprising an optical fibre, alternatively connects the first source 101 or the second source 102 to the handpiece 10, in order to convey the laser radiation thereto. In a manner known per se and not shown, the two sources 101, 102 can be combined with dichroic mirrors that allow the radiation of one or of the other of the two laser sources 101, 102 to be injected into the optical fibre 105, or other waveguide.

The cooling circuit 103 is operatively connected to the handpiece 10 by means of ducts 106 comprising a first pipe 106A for supplying cooling fluid to the handpiece 10 from the cooling circuit, and a second pipe 106B for return of the cooling fluid (which has extracted heat from the handpiece) to the cooling circuit 103. The cooling fluid is preferably in liquid form and is, for example, formed of water or a water-based mixture (for example, formed of water and methanol).

The electronic control unit 104 is operatively connected with the handpiece by means of wiring 107 that conveys the power supply and the electronic signals to the handpiece 10, thereby allowing operative communication between handpiece 10 and control unit 104.

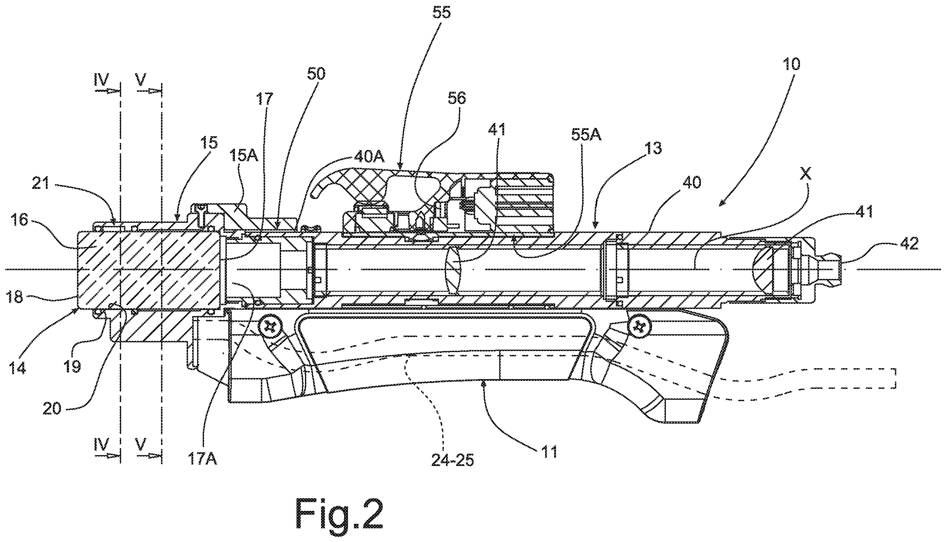

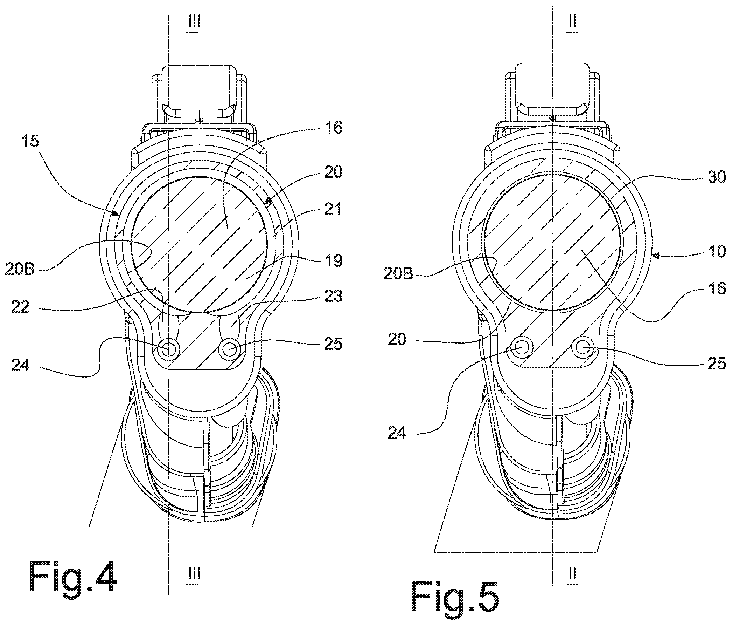

The handpiece 10 comprises a gripping structure 11, which forms at least part of the handle, a seat 12, housing an optical assembly 13 for focusing of the laser radiation coming from one of the laser sources 101, 102, and a cooling device of the epidermis 14 (see in particular Figs. 2 to 5), operatively connected to the cooling circuit 103 through the ducts 106.

The handpiece 10 of the present invention, by means of insertion of the suitable optical assembly 13, can be used to carry out dermatological and aesthetic treatments with differentiated wavelengths and parameters of use based on the clinical target concerned, such as, by way of non-limiting example: hair removal, reduction of wrinkles and skin rejuvenation, etc., on persons with skins of different phototype.

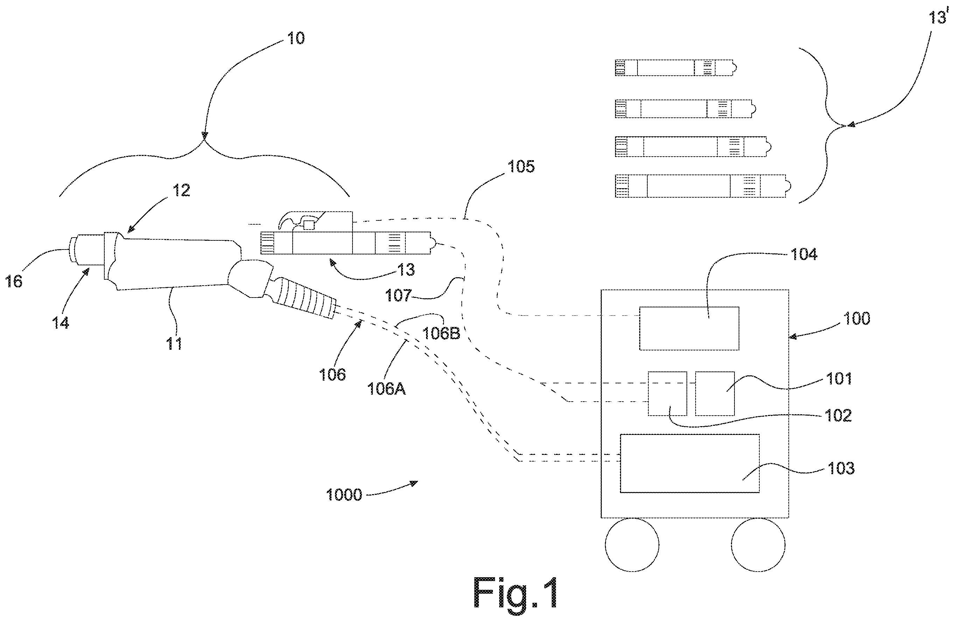

The optical assembly 13 is of the type rapidly interchangeable in the seat 12 with other optical assemblies to be used according to the type of treatment to be carried out. In Fig. 1, the optical assembly of the handpiece is shown external to the seat 12. The same figure also shows a kit of different optical assemblies that can be used in the handpiece, with different technical characteristics to one another, indicated as a whole with the number 13'.

As shown in greater detail in Figs. 2 to 5, the cooling device of the epidermis 14 comprises a sleeve 15, preferably made of metal, inside which a cooling body 16 is arranged, adapted to come into contact with the epidermis and, at the same time, to convey the laser beam coming from the optical assembly 13 toward the same area of the epidermis on which the cooling body 16 is in contact.

More in particular, the cooling body 16 comprises a rear surface 17 for entry of the laser beam, an opposite front surface 18 for contact with the epidermis and for exit of the laser beam, and a lateral surface 19 that extends between the front surface 18 and the rear surface 17.

A confined environment 17A is defined between the rear surface 17 of the cooling body and the optical assembly 13, which is exposed to air each time one optical assembly is replaced with another.

In this example, the cooling body 16 is a cylindrical body with two parallel faces or circular bases that correspond to the lateral surface 19. Naturally, other geometrical shapes are possible, such as prismatic shapes or other shapes with a circular, ellipsoidal or similar cross section.

The cooling body 16 is such as to allow the passage of the laser beam toward the epidermis, i.e., it is transparent, or at least partially transparent, to laser radiation. In this example, the cooling body 16 is made of sapphire, but other materials, such as fused quartz, optical glasses, etc., can be used as a function of the wavelength of the laser beam employed.

It should be noted that the shape of the cylinder that forms the cooling body 16 extends prevalently in an axial direction (axis indicated with X), i.e., in the direction of propagation of the laser beam. In other words, the axial dimension is greater than the transverse dimension (diameter), i.e., the cylinder is longer than it is wide.

In this example, the cooling body 16 projects from the sleeve 15, i.e., the front surface of the cooling body 16 is external to the sleeve.

The sleeve 15 comprises an inner surface 20 at least partially surrounding the lateral surface 19 of the cooling body 14. A circulation chamber 21 for the cooling fluid coming from the cooling circuit 103 is defined between the inner surface 20 of the sleeve 15 and the lateral surface 19 of the cooling body 16. The circulation chamber 21 at least partially surrounds the cooling body 16. The inner surface 20 of the sleeve 15 extends from the circulation chamber 21 toward the rear surface 17 of the cooling body 16.

As is apparent from the figures, the circulation chamber 21 is a space defined at least in part by the lateral surface of the cooling body and by the inner surface 20 of the sleeve 15.

The cooling fluid, circulating in the circulation chamber 21, is therefore in direct contact with the cooling body 16, thus allowing an optimal heat removal action, as explained in more detail below.

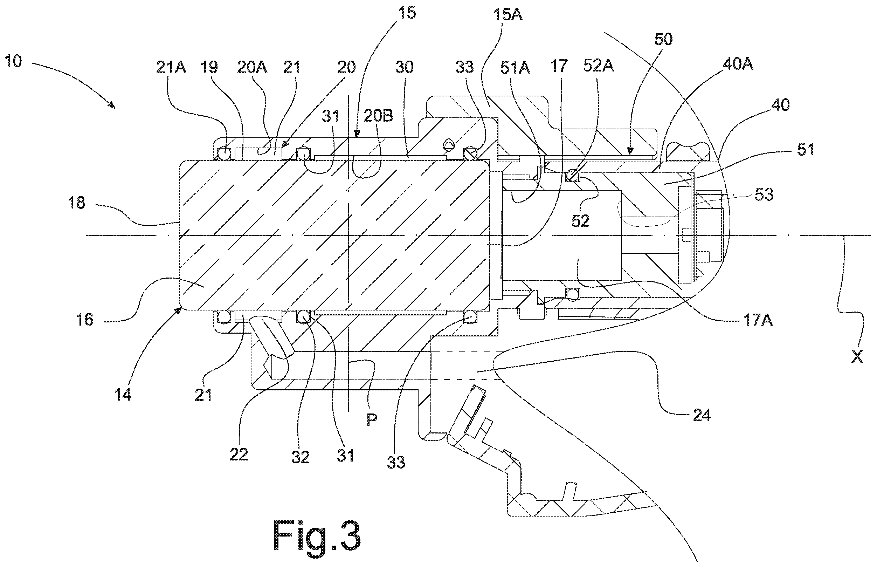

The cooling fluid circulation chamber 21 is positioned entirely between the front surface 18 of the cooling body 14 and a median plane P (Fig. 3), parallel to the front surface 18 and to the rear surface 17 of the cooling body 16, and equidistant from these surfaces. In examples of embodiment, the cooling fluid circulation chamber can also extend beyond the median plane P, toward the rear surface 17, although this is less advantageous, for the reasons clarified hereunder.

In practice, the circulation chamber 21 extends prevalently closer to the front surface 18 of the cooling body 16 with respect to the rear surface of the cooling body. In other terms, removal of heat from the cooling body takes place mostly close to the contact area with the epidermis with respect to the rear area of the cooling body 16, which is inside the handpiece.

A first sealing gasket 21A is arranged between the circulation chamber 21 and the front surface 18 of the cooling body 14.

The cooling fluid circulation chamber 21 comprises an inlet opening 22 of the cooling fluid located in an intermediate position between the front surface 18 and the rear surface 17 of the cooling body 16, and more preferably between the front surface 18 and the median plane P, so as to be closer to the front surface 18.

The cooling fluid circulation chamber 21 further comprises an outlet opening 23 of the cooling fluid from the circulation chamber 21, also located in an intermediate position between the front surface 18 and the rear surface 17 of the cooling body, and more preferably between the front surface 18 and the median plane P, so as to be closer to the front surface 18. For example,, the two openings 22 and 23 are arranged at a same axial position along the axis X.

A first duct 24 for supplying the cooling fluid to the circulation chamber 21 extends from the inlet opening 22, for example inside the gripping structure 11, and continues in the first pipe 106A connected, for supplying the handpiece, to the cooling circuit 103. Similarly, a second duct 25 for delivery of the cooling fluid from the circulation chamber extends from the outlet opening 23, for example inside the gripping structure 11, and continues in the second pipe 106B connected, for return from the handpiece, to the cooling circuit 103.

In this example, the sleeve 15 has a first portion 20A of the inner surface 20 that surrounds the cooling fluid circulation chamber 21, and a second portion 20B of the inner surface 20 that extends from the cooling fluid circulation chamber 21 toward the rear surface 17 of the cooling body 14. In particular, the second portion 20B of inner surface 20 has a greater extension in a radial direction with respect to the first portion 20A of inner surface, for example, at least double that of the first portion 20A. This second portion 20B defines a heat conduction interface between the inner surface 20 of the sleeve 15 and the cooling body 14.

In this example, the second portion 20B is provided with further chamber 30 containing cooling fluid, which has an annular cross section of smaller radial dimension with respect to the radial dimension of the cooling fluid circulation chamber 21.

This further chamber 30 is in fluid communication with the cooling fluid circulation chamber 21 through a more or less annular passage 31.

A sealing gasket 33 is positioned between the further chamber 30 and the rear face 17 of the cooling body 16.

As there is a passage 31 without sealing gasket between the chamber 21 and the further chamber 30, the cooling fluid that circulates in the circulation chamber 21 can pass from the circulation chamber 21 into the further chamber 30, filling it. Moreover, it has been found that the cooling fluid that fills the further chamber 30 does not circulate in this chamber, but is in static or quasi-static conditions and therefore does not have a heat removing effect. On the contrary, it forms a thermally conductive filling that facilitates heat transfer from the inner surface of the sleeve 15 to the outer surface of the cooling body.

In substance, the second portion 20B of the inner surface 20 of the sleeve 15 with the further chamber 30 defines a heat conduction interface between the inner surface 20 of the sleeve 15 and the cooling body 14.

In other embodiments, this heat conduction interface could also be formed by a different thermally conductive liquid to the cooling fluid and a sealing gasket could be inserted into the passage 31. In yet other embodiments, the conduction interface could consist of a solid filling material that fills the further chamber 30.

However, both these embodiments have some drawbacks, with regard to construction, as they make production of the device more complicated. Moreover, the solid interface would make the cooling body 16 difficult to disassemble, remove and replace.

Therefore, the embodiment illustrated, in which the further chamber 30 is filled with the same cooling fluid circulating in the circulation chamber 31 is particularly advantageous.

Both the first portion 20A and the second portion 20B of inner surface 20 of the sleeve 15 form a collection surface of laser radiation reflected and backscattered from the skin when the handpiece is in use. The backscattered laser radiation affects the portion 20A-20B of the inner surface 20 of the sleeve 15 and generates heat. Part of the heat is transferred by conduction through the static cooling fluid in the further chamber 30, toward the cooling body 16, generating a temperature gradient between the front surface 18 and the rear surface 17, for the purposes described hereunder.

The optical assembly 13 has an outer sheath 40 of elongated shape, which extends around a central axis thereof, in this example coincident with the axis X of the cooling body 16. The optical assembly 13 comprises internally one or more optical components 41 (such as, by way of non limiting example: mirrors, lenses, reflecting, diffractive or refractive elements) to allow the appropriate shape of the laser beam coming from one or more laser sources 101, 102 contained in the platform 100.

In the rear area the sheath 40 comprises a first connection interface 42 for the optical fibre or waveguide 105 for connection to the platform 100.

As mentioned, the optical assembly 13 is of interchangeable type. Therefore, a second interface 50 that forms a connection for the optical assembly 13 is present in the handpiece 10, at the housing seat 12 of the optical assembly.

For example, this second interface 50 comprises a bushing 51 with one end 51A screwed to a connection quill 15A of the sleeve 15. The connection thread between the end 51A of the bushing 51 and the sleeve 15 forms a thermally conductive connection.

The bushing 51 has, on its outer surface, an annular recess 52, coaxial to the axis X, i.e., to the axis of the optical assembly 13, in which a component 52A, for example a garter spring, is inserted to allow sealed coupling between the optical assembly 13 and the bushing 51. Coupling takes place by slidingly inserting the distal end 40A of the sheath 40 on the outer surface of the bushing 51 (see Fig. 3). The inside of the bushing 51 is facing the rear surface 17 of the cooling body and in practice delimits the confined environment 17A.

++In some embodiments, the bushing 51 comprises an inner annular boss 53 defining a collection surface of laser radiation backscattered from the skin during use of the handpiece. The annular boss 53 has a surface facing the cooling body 16 and preferably orthogonal to the axis X of the cooling body 16 and of the bushing 51.

An electronic control unit 55 of the handpiece 10, comprising a third interface 55A (see Fig. 2) for electrical/electronic connection with the wiring 107 for connection to the electronic control unit 104, is fixed on the sheath 40 of the optical assembly 13. The control unit 55 comprises one or more controls available to the user.

The electronic control unit 55 is associated with a specific optical assembly and comprises an electronic reference (indicated schematically with 56) for identification of said specific optical assembly, so that, by means of the connection to the electronic control unit 104, the latter knows which optical assembly is currently in use.

The handpiece 10 now described allows easy and rapid replacement of the optical assembly 13, so that the operator can rapidly select and insert those with characteristics suitable for the source and for the performances (spot, power, fluence etc.) to use for the treatment in question. With respect to conventional handpieces available on the market, the present invention allows the operator to switch much more easily from one treatment to another, where necessary also changing the operating wavelength, without the need to replace the whole handpiece, but simply by replacing a sub-unit thereof consisting of the optical assembly 13. Moreover, the handpiece 10 proposed is suitable for both static and dynamic use on the skin, thus making it possible for the operator to vary operation according to specific requirements.

Thanks to a delivery window of the laser beam formed by a cooling body 16 located in the front part of the handpiece 10, the latter operates in contact with the patient's skin, so that the feeling of comfort and coolness during treatment is optimal and long lasting.

The cooled window formed by the cooling body 16 also forms a solid and precise spacer that ensures that the characteristics of the laser on the tissue are always those selected by the operator, without the possibility of errors caused by incorrect distancing of the source from the skin.

The handpiece 10 is optimized so that the characteristics of the laser are greatly independent from environmental conditions such as external temperature and relative humidity, thus allowing maximum safety during use by the operator.

In fact, as already mentioned, when an optical assembly 13 is connected on the handpiece 10 damp air remains trapped in the volume 17A between the rear surface 17 of the cooling body 16 and the outermost optical component of the optical assembly 13. The humidity contained in this volume of air could condense on the rear surface 17 of the cooling body 16, forming lenticular masses of water, which could lead to a localized concentration of the laser beam, with the risk of causing pinpoint burns on the epidermis.

The architecture of the cooling device 14 of the handpiece 10 is however produced to obtain a temperature gradient along the axis X of the cooling body 16 such that, during use, the front surface 18 of the cooling body 16, which comes into contact with the epidermis of the area to be treated, is at an optimal minimum temperature for cooling of the epidermis, while the rear surface 17 of the cooling body 17 is at a higher temperature and such as to prevent the formation of condensation on the surface 17.

In substance, the arrangement described ensures that, in use, the temperature of the front surface 18 is sufficiently low to guarantee pain-free treatment for the patient, while the rear surface 17 is at a temperature above the condensation temperature, in any condition of humidity of the ambient air.

This effect is optimized by the fact that the inner walls around the area that the rear surface 17 of the cooling body 16 faces are structured so as to maximize reception of the radiation reflected and backscattered from the skin, i.e., maximize the heating obtainable, so that the temperature in this area is always above the condensation point (dew point) of the air.

In practice, it is useful to cool the handpiece so that cooling is concentrated on the front part of the cooling body in contact with the epidermis while this cooling is less on the rear part, where, in combination with the heat derived from the radiation reflected and backscattered from the skin, a temperature suitable to avoid condensation of humidity on the rear surface of the cooling body is obtained. In practice, the rear surface of the cooling body 16 must be at a greater temperature than the dew point temperature in the confined environment 17A, i.e., the temperature at which the water vapour trapped in the confined environment 17A condenses.

Therefore, the method of cooling the handpiece provides for, during emission of laser radiation through the cooling body, conditioning of the temperature on the rear surface 17 of the cooling body to a value greater than the dew point temperature relating to the space inside said confined environment 17A facing the rear surface of the cooling body,

For example, the temperature at which the rear surface 17 is conditioned is greater than 20°C and more preferably between 20°C and 40°C, for example, in relation to an emission power of the laser radiation between 20 and 100; preferably said light radiation is a laser radiation.

As said, conditioning of the rear surface 17 takes place by cooling the cooling body by making the cooling liquid circulate in the circulation chamber 21 around the cooling body 16 (removal of heat by the cooling fluid takes place prevalently in the part of cooling body 16 between the front surface 18 of the cooling body 16 and the median plane P) and, at the same time, also by heating the walls of the confined environment 17A facing the rear surface 17 by means of thermal conduction of heat coming from the sleeve heated by the laser radiation reflected and backscattered from the skin toward the inside of the handpiece and by the laser radiation reflected and backscattered from the skin directly against the walls of the confined environment.

Advantageously, with the handpiece according to the invention it is possible, for example, to use a cooling fluid, preferably a liquid, such as water or a mixture, that enters the handpiece 10 at a temperature between around 10°C and 24°C, and more preferably between 15°C and 22°C. With these temperature ranges it is possible to guarantee a temperature of the rear surface 17 of the cooling body 16 suitable to avoid condensation of humidity for a wide range of operating parameters of the handpiece (power, fluence, spot size of the laser), maintaining the temperature of the front surface 18 at a value suitable to cool the epidermis of the patient during the treatment laser.

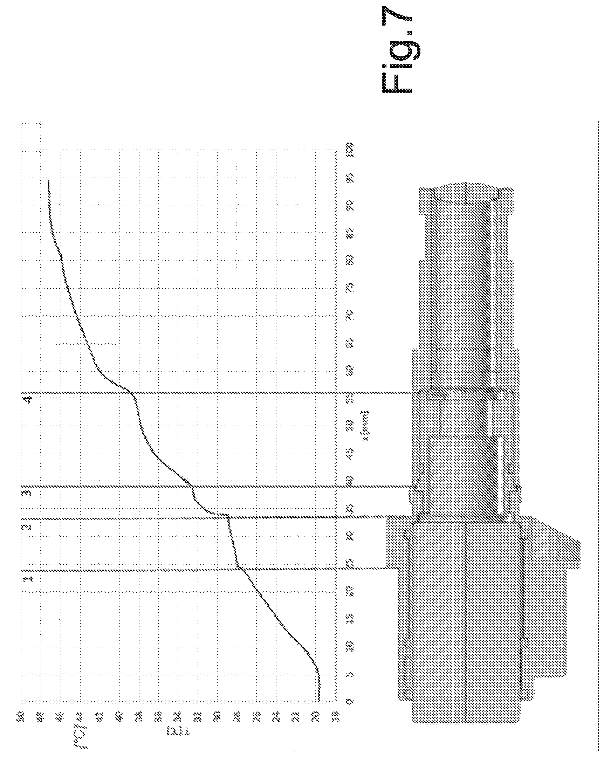

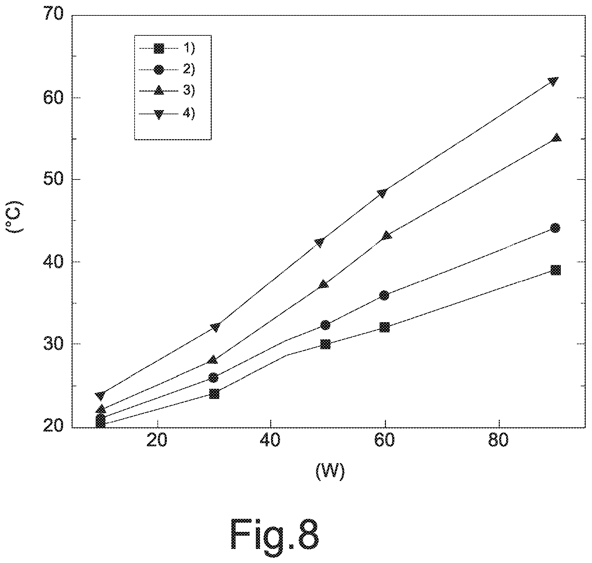

Figs. 7 and 8 show some experimental results validating the effectiveness of the architecture of the handpiece in question. Fig.7 shows a graph exhibiting the temperature measured along the profile of the external extrados of the handpiece, after 15 minutes of emission on a diffusing body (comparable to the skin) placed in contact with the front surface 18, in relation to a flow rate of cooling liquid entering the circulation chamber of 0.53l/min and temperature of 19°C, with laser emission power of 50W. Fig. 8 shows a graph of the temperature of the points of interest as a function of the power of the laser, with the same flow rate and temperature of the cooling liquid. In all the cases proposed, there was never any formation of condensation inside the confined environment.

The geometry of the handpiece 10, besides maximizing the temperature gradient in axial direction X along the cooling body 16, minimizes the radial gradient on both the front 18 and rear 17 surfaces, substantially guaranteeing the same properties on the whole of the area of the surfaces.

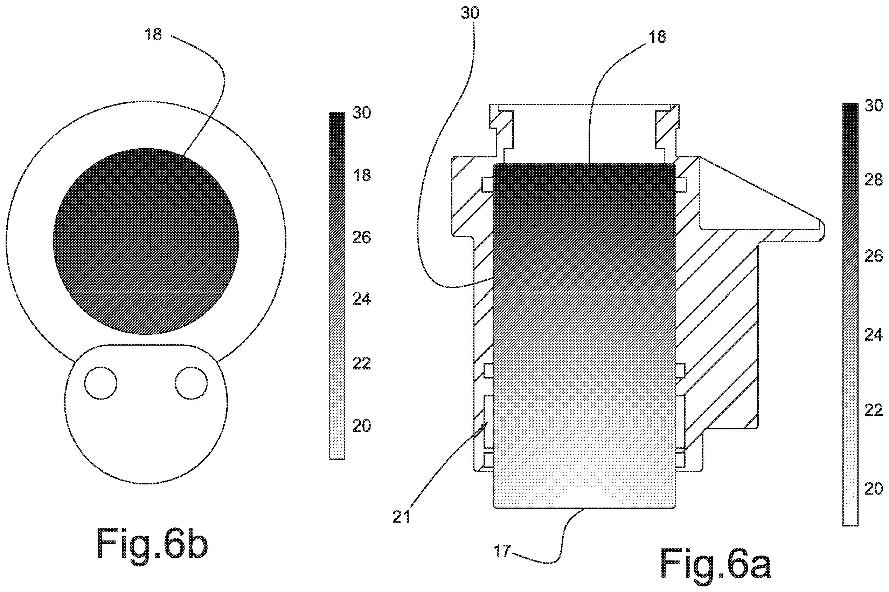

In this regard, Figs. 6a and 6b show a schematic section of the sleeve 15 with the cooling body 16, illustrating in greyscale the temperature trend (in relation to the same operating parameters of Fig. 7) along the axial and radial direction of the cooling body 16. The optimal distribution of the temperature is evident from Figs. 6a and 6b.

Advantageously, the architecture of the handpiece 10 is such that the external temperature of the handpiece as a whole is optimized, guaranteeing acceptable temperatures also at the end held by the operator, even in the case of prolonged use or with high power laser emission.

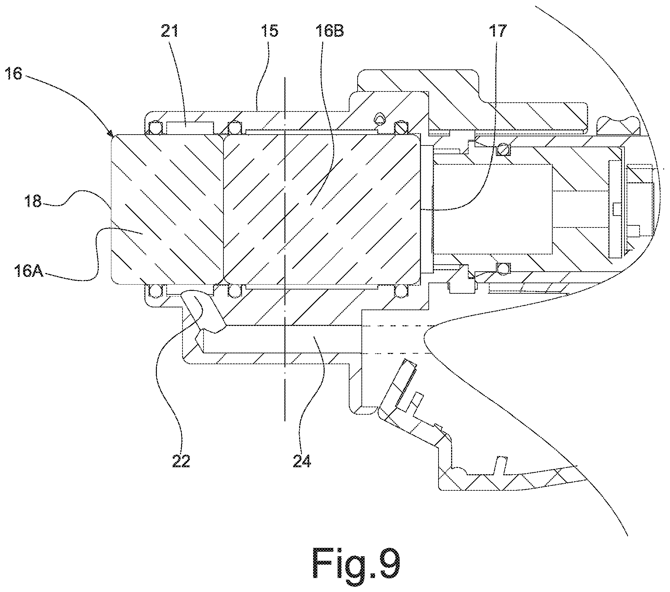

The construction of the cooling body can differ from the one indicated above. For example, Fig. 9 shows the case in which the cooling body is formed by two distinct pieces, for example, with the same cross section, arranged in contact with each other coaxially (if necessary with the interposition of layers useful to allow correct transmission of the light radiation), but made of different materials, for example with different coefficients of thermal conductivity. In this example, also the axial lengths can differ.

For example, the cooling body can be formed by a first piece made of sapphire 16A and a second piece 16B, made of "fused silica" or optical glass, wherein the axial length of the second piece 16B is preferably greater, and with the circulation chamber 21 that prevalently surrounds the first piece 16A.

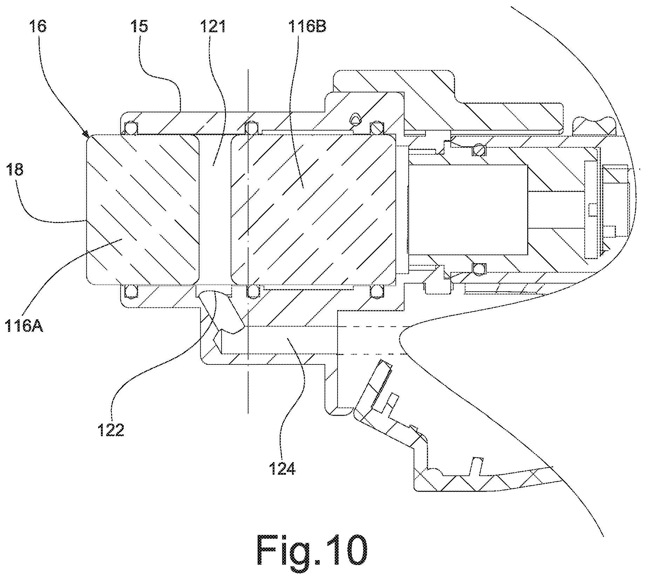

Fig. 10 shows the case in which the cooling body is formed by two distinct pieces (made of the same materials, for example sapphire, or of different materials, according to requirements) and separated from each other by a gap that forms the circulation chamber 121, in which the fluid enters through the opening 122 connected to the duct 124 and exits through an opening not shown in the figures. For example, the first piece 116A, the one defining the front surface 18 for contact with the epidermis, has a lower axial length with respect to the second piece 116B.

In other examples, the circulation chamber can also comprise a part that, in addition to the gap described above, surrounds the lateral surface of the first piece 116A and/or of the second piece 116B.

It is understood that the description illustrated only represents possible non-limiting embodiments of the invention, and that changes in form and arrangement may be mad without departing from the scope of the concept underlying the invention. Any reference numbers in the appended claims are provided purely in order to facilitate the reading thereof in the light of the foregoing description and of the accompany drawings and do not in any way limit the scope of protection.

## Claims

1. A handpiece (10) for treating the skin by means of light radiation; wherein the handpiece (10) comprises a gripping structure, a seat (12) for an interchangeable optical assembly (13), and a cooling device (14) of the epidermis; wherein the cooling device (14) comprises:
- a cooling body (16) for cooling the epidermis adapted for a light beam for treating the skin to pass through, the cooling body (16) being provided with an outer surface comprising:
a rear surface (17) for entry of the light beam; an opposite front surface (18) for contact with the epidermis and for exit of the light beam; and
a lateral surface provided between the front surface (18) and the rear surface (17);
a sleeve (15), in which the cooling body (16) is housed; wherein the sleeve (15) comprises an inner surface (20) at least partially surrounding the lateral surface of the cooling body (16);
- a circulation chamber (21) of a cooling fluid adapted to cool said cooling body (16), said chamber (21) being defined between the inner surface (20) of the sleeve (15) and at least one portion of the outer surface of the cooling body (16), wherein said circulation chamber (21) of a cooling fluid is defined, at least in part, between the inner surface (20) of the sleeve (15) and the lateral surface of the cooling body (16) and at least partially surrounds the cooling body (16);
- a first supply duct of the cooling fluid to the cooling fluid circulation chamber (21) and a second delivery duct of the cooling fluid from the cooling fluid circulation chamber (21), adapted to operatively connect the handpiece (10) to a cooling fluid cooling circuit.

2. The handpiece (10) of claim 1, wherein said cooling body (16) is made
- in one piece, or
- in at least two pieces arranged facing one another in the direction of passage of the light beam.

3. The handpiece (10) of claim 1 or 2, wherein said circulation chamber (21) of a cooling fluid is defined at least in part between said at least two pieces arranged facing one another in the direction of passage of the light beam.

4. The handpiece (10) of one or more of the preceding claims, wherein the cooling fluid circulation chamber (21) is positioned, at least in part, between the front surface (18) of the cooling body (16) and a median plane, equidistant from the front surface (18) and from the rear surface (17) of the cooling body (16); preferably the circulation chamber (21) comprises an inlet opening of the cooling fluid located between the front surface (18) of the cooling body (16) and the median plane; preferably the circulation chamber (21) comprising an outlet opening of the cooling fluid located between the front surface (18) of the cooling body (16) and the median plane; preferably the cooling fluid circulation chamber (21) is positioned entirely between the front surface (18) of the cooling body (16) and the median plane.

5. The handpiece (10) of one or more of the preceding claims, wherein the cooling body (16) has a greater axial dimension with respect to a transverse dimension, orthogonal to the axial dimension.

6. The handpiece (10) of one or more of the preceding claims, wherein the sleeve (15) comprises:
- a first portion (20A) of the inner surface (20) surrounding the cooling fluid circulation chamber (21); and
- a second portion (20B) of the inner surface extending from the cooling fluid circulation chamber (21) toward the rear surface (17) of the cooling body (16);
- wherein the second portion of inner surface (20B) has an extension in an axial direction greater than the first portion of inner surface (20A), preferably at least double that of the first portion of inner surface (20A); preferably the second portion of inner surface (20B) of the sleeve (15) defines a collection and reflection surface of light radiation reflected and back-scattered from the skin when the handpiece (10) is in use.

7. The handpiece (10) of one or more of the preceding claims, wherein a heat conduction interface between the inner surface (20) of the sleeve (15) and the cooling body (16) is defined between the cooling fluid circulation chamber (21) and the rear surface (17) of the cooling body (16); preferably the heat conduction interface comprises a further chamber (30) containing cooling fluid, preferably the further chamber (30) is in fluid communication with the cooling fluid circulation chamber (21) through a more or less annular passage (31); preferably the further chamber (30) has an annular cross section, of smaller radial dimension with respect to the radial dimension of the cooling fluid circulation chamber (21).

8. The handpiece (10) of one or more of the preceding claims, further comprising an interface (50) integral with the sleeve (15) and coaxial thereto; wherein the interface (50) forms a connection for the interchangeable optical assembly (13); preferably the interface (50) integral with the sleeve (15) comprises a bushing (51) with an end fixed to the sleeve (15), preferably through a heat conductive coupling, preferably a screw coupling; preferably the bushing (51) comprising an inner annular boss (53) defining a collection surface of light radiation backscattered from the skin during use of the handpiece (10).

9. The handpiece (10) of one or more of the preceding claims, wherein the optical assembly (13) comprises a connection interface adapted to be connected with a power conveying duct such as an optical fibre, a waveguide, or an electrical cable.

10. The handpiece (10) of one or more of the preceding claims, wherein said circulation chamber (21) is a space defined at least in part by the lateral surface of the cooling body (16) and by the inner surface (20) of said sleeve (15).

11. The handpiece (10) of one or more of the preceding claims, comprising a confined environment (17A) facing the rear surface (17) of the cooling body (16) and delimited at least in part by the optical assembly (13), wherein said confined environment (17A) is exposed to air each time one optical assembly (13) is replaced with another.

12. The handpiece (10) of one or more of the preceding claims comprising an electronic reference (56) for identification of the specific optical assembly (13) inserted in said seat (12).

13. A device for treating the skin by means of light radiation comprising a base platform (100) provided with an electronic control unit (104) of the device, a cooling circuit for a cooling fluid, and at least one power source, wherein
- said at least one power source is adapted to be operatively connected, through a power conveying duct (105), to a handpiece (10) according to one or more of the preceding claims,
- the cooling circuit (103) is adapted to be operatively connected to the handpiece (10) by means of a first pipe (106A) for supplying cooling fluid to the handpiece (10) from the cooling circuit, and a second pipe (106B) for return of cooling fluid from said handpiece (10) to the cooling circuit,
- the electronic control unit (104) is adapted to be operatively connected with the handpiece (10) by means of electrical wiring (107).

14. Method for cooling a handpiece (10) during a non-therapeutic and non-surgical procedure for treating skin, the handpiece (10) being of the type for treating the skin by means of light radiation, wherein said handpiece (10) comprises a gripping structure (11) and a seat (12) for an interchangeable optical assembly (13), and a cooling device (14) for cooling epidermis; wherein the cooling device (14) comprises:
- a cooling body (16) for cooling the epidermis adapted for a light beam for treating the skin to pass through, the cooling body (16) comprising:
a rear surface (17) for entry of the light beam;
an opposite front surface (18) for contact with the epidermis and for exit of the light beam; and
a lateral surface extending between the front surface (18) and the rear surface (17);
- a sleeve (15), in which the cooling body (16) is housed; wherein the sleeve (15) comprises an inner surface (20) at least partially surrounding the lateral surface of the cooling body (16);
- a circulation chamber (21) of a cooling fluid adapted to cool said cooling body (16), defined between the inner surface (20) of the sleeve (15) and at least one outer portion of the cooling body (16), wherein said circulation chamber (21) of a cooling fluid is defined, at least in part, between the inner surface (20) of the sleeve (15) and the lateral surface of the cooling body (16) and at least partially surrounds the cooling body (16);
- a first supply duct of the cooling fluid to the cooling fluid circulation chamber (21) and a second delivery duct of the cooling fluid from the cooling fluid circulation chamber (21), adapted to operatively connect the handpiece (10) to a cooling fluid cooling circuit;
and wherein the handpiece (10) comprises a confined environment (17A) facing said rear surface (17) of the cooling body (16) and delimited at least in part by the assembly (13),
said method providing for, during emission of light radiation through said cooling body (16), conditioning of the temperature on the rear surface (17) of said cooling body (16) to a value greater than the dew point temperature relating to the space in said confined environment (17A) facing the rear surface (17) of the cooling body (16).

15. Method for cooling a handpiece (10) according to claim 14, wherein
- - said rear surface (17) of said cooling body (16) is conditioned to a temperature greater than 20°C and more preferably of between 20°C and 40°C, and/or
- said light radiation has a power of between 20W and 100W; preferably said light radiation is a laser radiation.

16. Method for cooling a handpiece (10) according to one or more of claims 14 to 15, wherein the cooling device (14) of the handpiece (10) further comprises
- a sleeve (15), in which the cooling body (16) is housed; wherein the sleeve (15) comprises an inner surface (20) at least partially surrounding the lateral surface of the cooling body (16);
- a circulation chamber (21) of a cooling fluid defined between the inner surface (20) of the sleeve (15) and at least one outer portion of the cooling body (16), and wherein conditioning of the temperature of said rear surface (17) further provides for cooling of said cooling body (16) by means of circulation of cooling fluid in said circulation chamber (21); preferably said conditioning of the temperature of said rear surface (17) also provides for heating of the walls of said confined environment (17A) facing said rear surface (17) of the cooling body (16) by means of thermal conduction of heat coming from said sleeve (15) heated by the light radiation reflected and backscattered from the skin toward the inside of the handpiece (10) and of the light radiation reflected and backscattered from the skin directly against the walls of said confined environment (17A) facing the rear surface (17) of the cooling body (16); preferably said handpiece (10) further comprises an interface (50) integral with the sleeve (15) and coaxial thereto; wherein the interface (50) forms a connection for the interchangeable optical assembly (13), preferably the interface (50) integral with the sleeve (15) comprises a bushing (51) with an end fixed to the sleeve (15), preferably through a thermally conductive coupling, preferably a screw coupling; preferably the bushing (51) comprising an inner annular boss (53) defining a collection surface of light radiation backscattered from the skin during use of the handpiece (10).

## Patentansprüche

1. Handstück (10) zur Behandlung der Haut mittels Lichtstrahlung; wobei das Handstück (10) eine Griffstruktur, eine Aufnahme (12) für eine austauschbare optische Baugruppe (13) und eine Vorrichtung (14) zur Kühlung der Epidermis umfasst; wobei die Kühlvorrichtung (14) umfasst:
- einen Kühlkörper (16) zur Kühlung der Epidermis, der ausgebildet ist, dass ein Lichtstrahl zur Behandlung der Haut durch ihn hindurchtreten kann, wobei der Kühlkörper (16) eine Außenfläche bereitstellt, die umfasst:
∘ eine Rückseite (17) für den Eintritt des Lichtstrahls;
∘ eine gegenüberliegende Vorderseite (18) für den Kontakt mit der Epidermis und für den Austritt des Lichtstrahls; und
∘ eine Seitenfläche, die zwischen der Vorderseite (18) und der Rückseite (17) bereitgestellt ist;
∘ eine Hülse (15), die den Kühlkörper (16) aufnimmt; wobei die Hülse (15) eine Innenfläche (20) umfasst, die die Seitenfläche des Kühlkörpers (16) zumindest teilweise umgibt;
- eine Zirkulationskammer (21) für ein Kühlfluid, das zum Kühlen des Kühlkörpers (16) ausgebildet ist, wobei die Kammer (21) zwischen der Innenfläche (20) der Hülse (15) und mindestens einem Abschnitt der Außenfläche des Kühlkörpers (16) definiert ist, wobei die Zirkulationskammer (21) für ein Kühlfluid zumindest teilweise zwischen der Innenfläche (20) der Hülse (15) und der Seitenfläche des Kühlkörpers (16) definiert ist und den Kühlkörper (16) zumindest teilweise umgibt;
- einen ersten Zuführkanal für das Kühlfluid zur Kühlfluid-Zirkulationskammer (21) und einen zweiten Abführkanal für das Kühlfluid aus der Kühlfluid-Zirkulationskammer (21), die ausgebildet sind, das Handstück (10) operativ mit einem Kühlfluid-Kühlkreislauf zu verbinden.

2. Handstück (10) gemäß Anspruch 1, wobei der Kühlkörper (16)
- einteilig ausgebildet ist oder
- aus mindestens zwei Teilen ausgebildet ist, die in Durchgangsrichtung des Lichtstrahls einander gegenüberliegend angeordnet sind.

3. Handstück (10) gemäß Anspruch 1 oder 2, wobei die Zirkulationskammer (21) für ein Kühlfluid zumindest teilweise zwischen den mindestens zwei Teilen definiert ist, die in Durchgangsrichtung des Lichtstrahls einander gegenüberliegend angeordnet sind.

4. Handstück (10) gemäß einem oder mehreren der vorangegangenen Ansprüche, wobei die Kühlfluid-Zirkulationskammer (21) zumindest teilweise zwischen der Vorderseite (18) des Kühlkörpers (16) und einer Mittelebene angeordnet ist, die in gleichem Abstand zur Vorderseite (18) und zur Rückseite (17) des Kühlkörpers (16) liegt; vorzugsweise umfasst die Zirkulationskammer (21) eine Einlassöffnung für das Kühlfluid, die zwischen der Vorderseite (18) des Kühlkörpers (16) und der Mittelebene angeordnet ist; vorzugsweise umfasst die Zirkulationskammer (21) eine Auslassöffnung für das Kühlfluid, die zwischen der Vorderseite (18) des Kühlkörpers (16) und der Mittelebene angeordnet ist; vorzugsweise ist die Kühlfluid-Zirkulationskammer (21) vollständig zwischen der Vorderseite (18) des Kühlkörpers (16) und der Mittelebene angeordnet.

5. Handstück (10) gemäß einem oder mehreren der vorangegangenen Ansprüche, wobei der Kühlkörper (16) eine größere axiale Abmessung im Verhältnis zu einer Querabmessung aufweist, die orthogonal zur axialen Abmessung verläuft.

6. Handstück (10) gemäß einem oder mehreren der vorangegangenen Ansprüche, wobei die Hülse (15) umfasst:
- einen ersten Abschnitt (20A) der Innenfläche (20), der die Kühlfluid-Zirkulationskammer (21) umgibt; und
- einen zweiten Abschnitt (20B) der Innenfläche, der sich von der Kühlfluid-Zirkulationskammer (21) in Richtung der Rückseite (17) des Kühlkörpers (16) erstreckt;
- wobei der zweite Abschnitt der Innenfläche (20B) eine Ausdehnung in axialer Richtung aufweist, die größer ist als die des ersten Abschnitts der Innenfläche (20A), vorzugsweise mindestens doppelt so groß wie die des ersten Abschnitts der Innenfläche (20A); vorzugsweise definiert der zweite Abschnitt der Innenfläche (20B) der Hülse (15) eine Sammel- und Reflexionsfläche für Lichtstrahlung, die von der Haut reflektiert und zurückgestreut wird, wenn das Handstück (10) in Gebrauch ist.

7. Handstück (10) gemäß einem oder mehreren der vorangegangenen Ansprüche, wobei eine Wärmeleitungsschnittstelle zwischen der Innenfläche (20) der Hülse (15) und dem Kühlkörper (16) zwischen der Kühlfluid-Zirkulationskammer (21) und der Rückseite (17) des Kühlkörpers (16) definiert ist; vorzugsweise umfasst die Wärmeleitungsschnittstelle eine weitere Kammer (30), die Kühlfluid enthält; vorzugsweise steht die weitere Kammer (30) über einen mehr oder weniger ringförmigen Durchgang (31) in Fluidverbindung mit der Kühlfluid-Zirkulationskammer (21); vorzugsweise weist die weitere Kammer (30) einen ringförmigen Querschnitt auf, der eine kleinere radiale Abmessung aufweist als die radiale Abmessung der Kühlfluid-Zirkulationskammer (21).

8. Handstück (10) gemäß einem oder mehreren der vorangegangenen Ansprüche, des Weiteren umfassend eine Schnittstelle (50), die einstückig mit der Hülse (15) ausgebildet und koaxial zu dieser angeordnet ist; wobei die Schnittstelle (50) eine Verbindung für die austauschbare optische Baugruppe (13) bildet; vorzugsweise umfasst die mit der Hülse (15) einstückige Schnittstelle (50) eine Buchse (51), deren eines Ende an der Hülse (15) befestigt ist, vorzugsweise durch eine wärmeleitende Verbindung, vorzugsweise eine Schraubverbindung; wobei die Buchse (51) vorzugsweise einen inneren ringförmigen Vorsprung (53) aufweist, der eine Sammelfläche für von der Haut während der Verwendung des Handstücks (10) zurückgestreutes Licht bildet.

9. Handstück (10) gemäß einem oder mehreren der vorangegangenen Ansprüche, wobei die optische Baugruppe (13) eine Verbindungsschnittstelle umfasst, die dazu ausgebildet ist, mit einem Energieübertragungskanal wie beispielsweise einer optischen Faser, einem Wellenleiter oder einem elektrischen Kabel verbunden zu werden.

10. Handstück (10) gemäß einem oder mehreren der vorangegangenen Ansprüche, wobei die Zirkulationskammer (21) ein Raum ist, der zumindest teilweise durch die Seitenfläche des Kühlkörpers (16) und durch die Innenfläche (20) der Hülse (15) begrenzt ist.

11. Handstück (10) gemäß einem oder mehreren der vorangegangenen Ansprüche, das eine abgeschlossene Umgebung (17A) umfasst, die der Rückseite (17) des Kühlkörpers (16) zugewandt ist und zumindest teilweise durch die optische Baugruppe (13) begrenzt wird, wobei die abgeschlossene Umgebung (17A) jedes Mal der Luft ausgesetzt wird, wenn eine optische Baugruppe (13) durch eine andere ersetzt wird.

12. Handstück (10) gemäß einem oder mehreren der vorangegangenen Ansprüche, das eine elektronische Referenz (56) zur Identifizierung der spezifischen optischen Baugruppe (13) umfasst, die in den genannten Sitz (12) eingesetzt ist.

13. Vorrichtung zur Behandlung der Haut mittels Lichtstrahlung, umfassend eine Basisplattform (100), die mit einer elektronischen Steuereinheit (104) der Vorrichtung, einem Kühlkreislauf für ein Kühlfluid und mindestens einer Energiequelle versehen ist, wobei
- die mindestens eine Energiequelle so ausgebildet ist, dass sie über eine Energieübertragungsleitung (105) operativ mit einem Handstück (10) gemäß einem oder mehreren der vorangegangenen Ansprüche verbunden werden kann,
- der Kühlkreislauf (103) dazu ausgebildet ist, mittels einer ersten Leitung (106A) zur Zufuhr von Kühlflüssigkeit aus dem Kühlkreislauf zum Handstück (10) und einer zweiten Leitung (106B) zum Rückfluss von Kühlflüssigkeit vom Handstück (10) mit dem Handstück (10) wirkverbunden zu werden,
- die elektronische Steuereinheit (104) so ausgebildet ist, dass sie mittels elektrischer Verkabelung (107) mit dem Handstück (10) wirkverbunden werden kann.

14. Verfahren zum Kühlen eines Handstücks (10) während eines nicht-therapeutischen und nicht-chirurgischen Verfahrens zur Behandlung der Haut, wobei das Handstück (10) von der Art ist, die die Haut mittels Lichtstrahlung behandelt, wobei das Handstück (10) eine Griffstruktur (11) und einen Sitz (12) für eine austauschbare optische Baugruppe (13) umfasst, sowie eine Kühlvorrichtung (14) zum Kühlen der Epidermis; wobei die Kühlvorrichtung (14) umfasst:
- einen Kühlkörper (16) zum Kühlen der Epidermis, der ausgebildet ist, dass ein Lichtstrahl zur Behandlung der Haut hindurchtreten kann, wobei der Kühlkörper (16) umfasst:
∘ eine Rückseite (17) für den Eintritt des Lichtstrahls;
∘ eine gegenüberliegende Vorderseite (18) für den Kontakt mit der Epidermis und für den Austritt des Lichtstrahls; und
∘ eine Seitenfläche, die sich zwischen der Vorderseite (18) und der Rückseite (17) erstreckt;
- eine Hülse (15), die den Kühlkörper (16) aufnimmt; wobei die Hülse (15) eine Innenfläche (20) umfasst, die die Seitenfläche des Kühlkörpers (16) zumindest teilweise umgibt;
- eine Zirkulationskammer (21) für ein Kühlfluid, das zum Kühlen des Kühlkörpers (16) ausgebildet ist, die zwischen der Innenfläche (20) der Hülse (15) und mindestens einem Außenabschnitt des Kühlkörpers (16) definiert ist, wobei die Zirkulationskammer (21) für ein Kühlfluid zumindest teilweise zwischen der Innenfläche (20) der Hülse (15) und der Seitenfläche des Kühlkörpers (16) definiert ist und den Kühlkörper (16) zumindest teilweise umgibt;
- einen ersten Zuführkanal für das Kühlfluid zur Kühlfluid-Zirkulationskammer (21) und einen zweiten Abführkanal für das Kühlfluid aus der Kühlfluid-Zirkulationskammer (21), die dazu ausgebildet sind, das Handstück (10) operativ mit einem Kühlfluid-Kühlkreislauf zu verbinden;
und wobei das Handstück (10) eine abgeschlossene Umgebung (17A) umfasst, die der genannten Rückseite (17) des Kühlkörpers (16) zugewandt ist und zumindest teilweise durch die Baugruppe (13) begrenzt wird,
wobei das Verfahren während der Emission von Lichtstrahlung durch den Kühlkörper (16) eine Konditionierung der Temperatur an der Rückseite (17) des Kühlkörpers (16) auf einen Wert bereitstellt, der größer ist als die Taupunkttemperatur, die sich auf den Raum in der abgeschlossenen Umgebung (17A) bezieht, die der Rückseite (17) des Kühlkörpers (16) zugewandt ist.

15. Verfahren zum Kühlen eines Handstücks (10) gemäß Anspruch 14, wobei
- die Rückseite (17) des Kühlkörpers (16) auf eine Temperatur von mehr als 20 °C und vorzugsweise zwischen 20 °C und 40 °C konditioniert wird, und/oder
- die Lichtstrahlung eine Leistung zwischen 20 W und 100 W aufweist; vorzugsweise ist die Lichtstrahlung eine Laserstrahlung.

16. Verfahren zum Kühlen eines Handstücks (10) gemäß einem oder mehreren der Ansprüche 14 bis 15, wobei die Kühlvorrichtung (14) des Handstücks (10) des Weiteren umfasst
- eine Hülse (15), die den Kühlkörper (16) aufnimmt; wobei die Hülse (15) eine Innenfläche (20) umfasst, die die Seitenfläche des Kühlkörpers (16) zumindest teilweise umgibt;
- eine Zirkulationskammer (21) für ein Kühlfluid, die zwischen der Innenfläche (20) der Hülse (15) und mindestens einem äußeren Abschnitt des Kühlkörpers (16) definiert ist,
und wobei die Temperaturregelung der genannten Rückseite (17) des Weiteren eine Kühlung des genannten Kühlkörpers (16) mittels Zirkulation des Kühlfluids in der genannten Zirkulationskammer (21) bereitstellt; vorzugsweise stellt die Temperaturregelung der Rückseite (17) auch eine Heizung der Wände der begrenzten Umgebung (17A) bereit, die der Rückseite (17) des Kühlkörpers (16) zugewandt sind, mittels Wärmeleitung von Wärme, die von der Hülse (15) stammt, die durch die von der Haut reflektierte und in Richtung des Inneren des Handstücks zurückgestreute Lichtstrahlung erwärmt wird (10) sowie der von der Haut reflektierten und zurückgestreuten Lichtstrahlung, die direkt gegen die Wände des begrenzten Raums (17A) trifft, die der Rückseite (17) des Kühlkörpers (16) zugewandt sind; vorzugsweise umfasst das Handstück (10) des Weiteren eine Schnittstelle (50), die einstückig mit der Hülse (15) ausgebildet und koaxial zu dieser angeordnet ist; wobei die Schnittstelle (50) eine Verbindung für die austauschbare optische Baugruppe (13) bildet, vorzugsweise umfasst die mit der Hülse (15) einstückige Schnittstelle (50) eine Buchse (51), deren eines Ende an der Hülse (15) befestigt ist, vorzugsweise durch eine wärmeleitende Verbindung, vorzugsweise eine Schraubverbindung; wobei die Buchse (51) vorzugsweise einen inneren ringförmigen Vorsprung (53) aufweist, der eine Sammelfläche für die während der Verwendung des Handstücks (10) von der Haut rückgestreute Lichtstrahlung definiert.

## Revendications

1. Une pièce à main (10) pour le traitement de la peau par rayonnement lumineux, dans laquelle la pièce à main (10) comprend une structure de préhension, un logement (12) pour un ensemble optique interchangeable (13) et un dispositif de refroidissement (14) de l'épiderme, le dispositif de refroidissement (14) comprenant :
- un corps de refroidissement (16) pour refroidir l'épiderme, conçu pour qu'un faisceau lumineux pour traiter la peau puisse le traverser, le corps de refroidissement (16) étant pourvu d'une surface externe comprenant :
une surface arrière (17) pour l'entrée du faisceau lumineux ; une surface avant opposée (18) pour le contact avec l'épiderme et pour la sortie du faisceau lumineux ; et
une surface latérale prévue entre la surface avant (18) et la surface arrière (17) ;
un manchon (15), dans lequel le corps de refroidissement (16) est logé ; ce manchon (15) comprenant une surface interne (20) entourant au moins partiellement la surface latérale du corps de refroidissement (16) ;
- une chambre de circulation (21) d'un fluide de refroidissement apte à refroidir ledit corps de refroidissement (16), cette chambre étant formée entre la surface interne (20) du manchon (15) et au moins une partie de la surface externe du corps de refroidissement (16), ladite chambre de circulation (21) d'un fluide de refroidissement étant formée, au moins en partie, entre la surface interne (20) du manchon (15) et la surface latérale du corps de refroidissement (16) et entourant au moins partiellement le corps de refroidissement (16) ;
- un premier conduit d'alimentation du fluide de refroidissement à la chambre de circulation de fluide de refroidissement (21) et un deuxième conduit de délivrance du fluide de refroidissement depuis la chambre de circulation de fluide de refroidissement (21), apte à connecter fonctionnellement la pièce à main (10) à un circuit de refroidissement de fluide de refroidissement.

2. La pièce à main (10) selon la revendication 1, dans laquelle ledit corps de refroidissement (16) est réalisé
- en une seule pièce ou
- en au moins deux pièces agencées face à face dans la direction de passage du faisceau lumineux.

3. La pièce à main (10) selon la revendication 1 ou 2, dans laquelle ladite chambre de circulation (21) de fluide de refroidissement est formée en partie entre lesdites deux pièces au moins agencées face à face dans la direction de passage du faisceau lumineux.

4. La pièce à main (10) selon l'une ou plusieurs des revendications précédentes, dans laquelle la chambre de circulation de fluide de refroidissement (21) est positionnée, au moins en partie, entre la surface avant (18) du corps de refroidissement (16) et un plan médian, équidistant de la surface avant (18) et de la surface arrière (17) du corps de refroidissement (16) ; de préférence la chambre de circulation (21) comprend une ouverture d'entrée du fluide de refroidissement située entre la surface avant (18) du corps de refroidissement (16) et le plan médian ; de préférence la chambre de circulation (21) comprend une ouverture de sortie du fluide de refroidissement située entre la surface avant (18) du corps de refroidissement (16) et le plan médian ; de préférence la chambre de circulation de fluide de refroidissement (21) est positionnée entièrement entre la surface avant (18) du corps de refroidissement (16) et le plan médian.

5. La pièce à main (10) selon l'une ou plusieurs des revendications précédentes, dans laquelle le corps de refroidissement (16) a une dimension axiale plus grande que sa dimension transversale, perpendiculaire à la dimension axiale.

6. La pièce à main (10) selon l'une ou plusieurs des revendications précédentes, dans laquelle le manchon (15) comprend :
- une première partie (20A) de la surface interne (20) entourant la chambre de circulation de fluide de refroidissement (21) ; et
- une deuxième partie (20B) de la surface interne s'étendant depuis la chambre de circulation de fluide de refroidissement (21) vers la surface arrière (17) du corps de refroidissement (16) ;
- la deuxième partie de la surface interne (20B) ayant une extension dans une direction axiale supérieure à la première partie de la surface interne (20A), de préférence au moins deux fois supérieure à la première partie de la surface interne (20A) ; la deuxième partie de la surface interne (20B) du manchon (15) formant de préférence une surface de collecte et de réflexion de rayonnement lumineux réfléchi et rétrodiffusé depuis la peau lorsque la pièce à main (10) est en cours d'utilisation.

7. La pièce à main (10) selon l'une ou plusieurs des revendications précédentes, dans laquelle une interface de conduction thermique entre la surface interne (20) du manchon (15) et le corps de refroidissement (16) est formée entre la chambre de circulation de fluide de refroidissement (21) et la surface arrière (17) du corps de refroidissement (16) ; l'interface de conduction thermique comprenant de préférence une chambre supplémentaire (30) contenant du fluide de refroidissement, cette chambre supplémentaire (30) étant de préférence en communication fluidique avec la chambre de circulation de fluide de refroidissement (21) grâce à un passage plus ou moins annulaire (31) ; la chambre supplémentaire (30) ayant de préférence une section transversale annulaire, de dimension radiale inférieure à la dimension radiale de la chambre de circulation de fluide de refroidissement (21).

8. La pièce à main (10) selon l'une ou plusieurs des revendications précédentes, comprenant en outre une interface (50) solidaire du manchon (15) et coaxiale avec celui-ci ; l'interface (50) formant une connexion pour l'ensemble optique interchangeable (13) ; l'interface (50) solidaire du manchon (15) comprenant de préférence une douille (51) avec une extrémité fixée au manchon (15), de préférence par l'intermédiaire d'un couplage thermiquement conducteur, de préférence une couplage par vis ; la douille (51) comprenant de préférence une bosse annulaire interne (53) formant une surface de collecte de rayonnement lumineux rétrodiffusé depuis la peau durant l'utilisation de la pièce à main (10).

9. La pièce à main (10) selon l'une ou plusieurs des revendications précédentes, dans laquelle l'ensemble optique (13) comprend une interface de connexion apte à être connectée à un conduit de transmission d'énergie tel qu'une fibre optique, un guide d'ondes ou un câble électrique.

10. La pièce à main (10) selon l'une ou plusieurs des revendications précédentes, dans laquelle ladite chambre de circulation (21) est un espace formé au moins en partie par la surface latérale du corps de refroidissement (16) et la surface interne (20) dudit manchon (15).

11. La pièce à main (10) selon l'une ou plusieurs des revendications précédentes, comprenant un environnement confiné (17A) faisant face à la surface arrière (17) du corps de refroidissement (16) et délimité au moins en partie par l'ensemble optique (13), ledit environnement confiné (17A) étant exposé à l'air à chaque fois que l'ensemble optique (13) est remplacé par un autre.

12. La pièce à main (10) selon l'une ou plusieurs des revendications précédentes, comprenant une référence électronique (56) pour l'identification de l'ensemble optique spécifique (13) inséré dans ledit logement (12).

13. Un dispositif pour le traitement de la peau par rayonnement lumineux comprenant une plateforme de base (100) pourvue d'une unité de commande électronique (104) du dispositif, un circuit de refroidissement pour un fluide de refroidissement et au moins une source d'alimentation, dans lequel :
- ladite ou lesdites source(s) d'alimentation est(sont) apte(s) à être connectée(s) fonctionnellement, par l'intermédiaire d'un conduit de transmission d'énergie (105), à une pièce à main (10) selon l'une ou plusieurs des revendications précédentes,
- le circuit de refroidissement (103) est apte à être connecté fonctionnellement à la pièce à main (10) au moyen d'un premier tuyau (106A) pour l'amenée du fluide de refroidissement à la pièce à main (10) depuis le circuit de refroidissement, et un deuxième tuyau (106B) pour le retour du fluide de refroidissement de ladite pièce à main (10) au circuit de refroidissement,
- l'unité de commande électronique (104) est apte à être connectée fonctionnellement à la pièce à main (10) au moyen d'un câblage électrique (107).

14. Procédé de refroidissement d'une pièce à main (10) durant une opération non thérapeutique et non chirurgicale de traitement de la peau, la pièce à main (10) étant du type pour traitement de la peau par rayonnement lumineux, dans lequel ladite pièce à main (10) comprend une structure de préhension (11) et un logement (12) pour un ensemble optique interchangeable (13), et un dispositif de refroidissement (14) pour refroidir l'épiderme ; dans lequel le dispositif de refroidissement (14) comprend :
- un corps de refroidissement (16) pour refroidir l'épiderme conçu pour qu'un faisceau lumineux pour traiter la peau puisse le traverser, le corps de refroidissement (16) comprenant :
une surface arrière (17) pour l'entrée du faisceau lumineux ;
une surface avant opposée (18) pour un contact avec l'épiderme et pour la sortie du faisceau lumineux ; et
une surface latérale s'étendant entre la surface avant (18) et la surface arrière (17) ;
- un manchon (15), dans lequel le corps de refroidissement (16) est logé ; le manchon (15) comprenant une surface interne (20) entourant au moins partiellement la surface latérale du corps de refroidissement (16) ;
- une chambre de circulation (21) d'un fluide de refroidissement apte à refroidir ledit corps de refroidissement (16), formée entre la surface interne (20) du manchon (15) et au moins une partie externe du corps de refroidissement (16), ladite chambre de circulation (21) d'un fluide de refroidissement étant formée, au moins en partie, entre la surface interne (20) du manchon (15) et la surface latérale du corps de refroidissement (16) et entourant au moins partiellement le corps de refroidissement (16) ;
- un premier conduit d'alimentation du fluide de refroidissement à la chambre de circulation de fluide de refroidissement (21) et un deuxième conduit de délivrance du fluide de refroidissement depuis la chambre de circulation du fluide de refroidissement (21), apte à connecter fonctionnellement la pièce à main (10) à un circuit de refroidissement de fluide de refroidissement ;
et dans lequel la pièce à main (10) comprend un environnement confiné (17A) faisant face à ladite surface arrière (17) du corps de refroidissement (16) et délimité au moins en partie par l'ensemble (13) ;
ledit procédé prévoyant, durant l'émission d'un rayonnement lumineux à travers ledit corps de refroidissement (16), de porter la température sur la surface arrière (17) dudit corps de refroidissement (16) à une valeur supérieure à la température du point de rosée relatif à l'espace dans ledit environnement confiné (17A) faisant face à la surface arrière (17) du corps de refroidissement (16).

15. Procédé de refroidissement d'une pièce à main (10) selon la revendication 14, dans lequel :
- ladite surface arrière (17) dudit corps de refroidissement (16) est portée à une température supérieure à 20°C et plus préférablement comprise entre 20°C et 40°C et/ou
- ledit rayonnement lumineux a une puissance comprise entre 20 et 100 W ; de préférence ledit rayonnement lumineux est une radiation laser.

16. Procédé de refroidissement d'une pièce à main (10) selon l'une ou plusieurs des revendications 14 à 15, dans lequel le dispositif de refroidissement (14) de la pièce à main (10) comprend en outre
- un manchon (15) dans lequel le corps de refroidissement (16) est logé ; le manchon (15) comprenant une surface interne (20) entourant au moins partiellement la surface latérale du corps de refroidissement (16) ;
- une chambre de circulation (21) d'un fluide de refroidissement formée entre la surface interne (20) du manchon (15) et au moins une partie externe du corps de refroidissement (16),
et dans lequel l'action sur la température de ladite surface arrière (17) réalise en outre le refroidissement dudit corps de refroidissement (16) grâce à la circulation du fluide de refroidissement dans ladite chambre de circulation (21) ; ladite action sur la température de ladite surface arrière (17) réalisant aussi de préférence le chauffage des parois dudit environnement confiné (17A) faisant face à ladite surface arrière (17) du corps de refroidissement (16) par conduction thermique de la chaleur provenant dudit manchon (15) chauffé par le rayonnement lumineux réfléchi et rétrodiffusé depuis la peau vers l'intérieur de la pièce à main (10) et du rayonnement lumineux réfléchi et rétrodiffusé depuis la peau directement contre les parois dudit environnement confiné (17A) faisant face à la surface arrière (17) du corps de refroidissement (16) ; ladite pièce à main (10) comprenant en outre de préférence une interface (50) solidaire du manchon (15) et coaxiale avec celui-ci ; l'interface (50) formant une connexion pour l'ensemble optique interchangeable (13), l'interface (50) solidaire du manchon (15) comprenant de préférence une douille (51) avec une extrémité fixée au manchon (15), de préférence par l'intermédiaire d'un couplage thermiquement conducteur, de préférence un couplage par vis ; la douille (51) comprenant de préférence une bosse annulaire interne (53) formant une surface de collecte de rayonnement lumineux rétrodiffusé depuis la peau durant l'utilisation de la pièce à main (10).
